# EUROPEAN PATENT SPECIFICATION

(11) **EP 0 666 985 B1**
(45) Date of publication and mention of the grant of the patent: **16.07.1997**
(21) Application number: 94900497.2
(22) Date of filing: 02.11.1993
(51) Int. Cl.: G01N 33/487, G01N 33/15

(54) **COMPACTION ASSAY FOR ASSESSMENT OF RESPIRATORY DISEASE THERAPY**
VERDICHTUNGSPRÜFUNG ZUR BEWERTUNG EINER THERAPIE FÜR EINE ATMUNGSKRANKHEIT
TITRAGE DE COMPACITE DETERMINANT L'EFFICACITE DE LA THERAPIE DES MALADIES RESPIRATOIRES

(30) Priority: 02.11.1992 US 971019; 06.10.1993 US 132681
(43) Date of publication of application: 16.08.1995
(73) Proprietor: GENENTECH, INC., South San Francisco, CA 94080-4990 (US)
(72) Inventor: DAUGHERTY, Ann, L., Palo Alto, CA 94301 (US); MRSNY, Randy, J., Redwood City, CA 94061 (US); PATAPOFF, Thomas, W., Belmont, CA 94002 (US)
(74) Representative: Walton, Seán Malcolm
(86) International application number: US9310519
(87) International publication number: WO9410567

(56) References cited:
- US-A- 3 914 985
- US-A- 4 072 045
- DATABASE WPI Week 7541, Derwent Publications Ltd., London, GB; AN 75-L1806W & SU,A,460 868 (WORK HYGIENE PROFES) 9 April 1975
- Am Rev Respiratory Disease 104 (1971) 88

## Description

### Field of the Invention

The present invention relates to an assay that measures the compaction of sputum samples of mammalian patients subject to respiratory disease associated with infected airway secretions.

The present invention relates to a compaction assay that measures the potential of a therapeutic to improve lung function in a patient subject to respiratory disease.

The present invention specifically relates to a compaction assay that is predictive of DNase therapeutic efficacy in a population of patients.

### Description of Related Art

### Respiratory Disease

Lung secretions are complex non homogenous materials that form a viscous hydrophilic gel. These secretions play an important role in the normal functioning of the respiratory airways. Respiratory diseases associated with infected airway secretions such as Cystic Fibrosis (CF), Bronchitis, and Pneumonia are characterized by purulent secretions that play a major part in the respiratory dysfunction associated with respiratory disease.

The viscosity of uninfected lung secretions has been attributed to mucus glycoproteins, whereas the viscosity of infected or purulent lung secretions has been attributed to mucus proteins and DNA (Shak *et al.,* PNAS 87:9188-9192 [1990]) and glycoproteins, DNA, proteins, lipids and cations, (Yeates, DB, The Lung: Scientific Foundations editor Crystal *et al.,* publisher Raven Press pg 197-203 [1991]).

Cystic fibrosis (CF) is a genetic disease characterized by a defect in secretory epithelia involved with electrogenic chloride transport and is the most common lethal genetic disease among Caucasians, being observed in approximately one in 2,500 live births (Boat *et al.,* The Metabolic Basis of Inherited Disease editors Scriver *et al.,* publisher McGraw Hill, pgs. 2649-2860 [1989]). Disturbances in the function of secretory epithelia result in several abnormalities including reduced pancreatic enzyme secretion, malabsorption in the gastrointestinal tract, and excessive secretion of bronchial mucus (Kerem *et al.* New England Journal of Medicine 323:1517-1522 [1990]). These excessive bronchial secretions provide an environment that supports chronic lung infections by opportunistic pathogens such as Pseudomonas aeruginosa (George, RH Arch Dis Child 62:438-439 [1987]). Large numbers of inflammatory leukocytes enter these infection sites and lyse after a short time, releasing their nuclear deoxyribonucleic acid (DNA) into the bronchial secretions. Extremely purulent secretions, which have been shown to contain as much as 15 mg/ml DNA (Matthews *et al.,* Am Rev Respir Dis 88:199-204 [19631), become too thick for the CF patient to clear; resulting in respiratory distress and progressive lung destruction (Welsh *et al.*, J Clin Invest 80:1523-1526 [1987]).

Pneumonia is characterized by inflammation of the lung parenchyma. Most cases of pneumonia are due to infection by bacteria or viruses, a few to inhalation of chemicals or trauma to the chest wall, and a small minority to rickettsias, fungi, and yeasts. The distribution of inflammation may be lobar, segmental, or lobular. One clinical manifestation of pneumonia is the presence of purulent secretions in the respiratory airways.

Bronchitis is characterized by inflammation of the mucus membrane of the bronchial tubes. There are many forms of bronchitis, including chronic bronchitis, hemorrhagic bronchitis, fibrinous bronchitis, capillary bronchitis, and asthmatic bronchitis. Chronic bronchitis is a condition of the bronchial tree characterized by cough, hypersecretion of mucus, and expectoration of sputum over a long period of time, associated with increased vulnerability to bronchial infection. An increase in the viscosity of purulent secretions is correlated with difficulty in expectoration in patients with chronic bronchitis (Dulfano *et al.,* Am. Rev. Respir. Dis. 104:88-98 [1971]).

Several approaches have been taken in the past to reduce the viscoelastic nature of purulent tracheobronchial secretions of mammalian patients subject to respiratory disease associated with infected airway secretions, hopefully to improve clearance of this material by the patient. Therapies such as the inhalation of water (Rosenbluth *et al.,* Archives of Disease in Childhood 49: pg 606-610 [1974]) and the use of mucolytics such as n-acetylcysteine (Mucomyst®) have not been successful.

Bovine pancreatic DNase I (Dornase) was shown to be effective in the treatment of pneumonia (Clifton, *et al.,* Cancer, (14):414-420 [1961]) but its use fell from favor due to irritation problems found to occur in humans using this bovine protein (Shak *et al.,* PNAS 87:9188 [1990]). Recently, Hubbard *et al.,* NEJM, 326:812-815 [1992]) and Aitken *et al.,* (JAMA vol. 267 pg. 1947-1951 [1992]) reported that human DNase cleaved high-molecular-weight DNA in purulent secretions in the airways, thereby reducing the viscoelasticity of the purulent secretions and improving lung function. Human DNase has also been indicated in the treatment of chronic bronchitis bronchiectasis, sinus infections and other respiratory disease (WO90/07572 published 12 July 1990).

Other therapeutics such as, anti-neutrophil elastase agents and secretory leucoprotease inhibitor are potentially promising new therapies for the treatment of CF (Mulherin *et al.,* Dig Dis 10(1) pg 29-37 [1992]). Antibiotic therapy is indicated for the treatment of chronic respiratory tract infections associated with CF and other respiratory disease (Mulherin *et al.,* Ir J Med Sci 160(6) pg 173-175 [1991] and de Montalembert *et al.,* Ann Pediatric 38(8) p523-528 [1991]). Chest physiotherapy has also been indicated as a mechanical approach to antiinfective therapy in cystic fibrosis (Zach *et al.,* Infection 15(5) pg. 381-384 [1987]) and asthma, chronic bronchitis, and bronchiectasis (Eid, *et al.,* Respir Care 36(4) pg. 270-282 [1991]).

### DNase

DNase is a phosphodiesterase capable of hydrolysing polydeoxyribonucleic acid. It acts extensively and non-specifically to degrade DNA and in this regard is distinguished from the relatively limited and sequence-specific restriction endonucleases. DNase I has a pH optimum near neutrality, an obligatory requirement for divalent cations, and produces 5' phosphate nucleotides on hydrolysis of DNA. DNase II exhibits an acid pH optimum, can be activated by divalent cations and produces 3'-phosphate nucleotides on hydrolysis of DNA. Multiple molecular forms of DNase I and DNase II are known.

DNase has a number of known utilities, and has been used for therapeutic purposes. Its principal therapeutic use has been to reduce the viscoelastic properties of infected airway secretions in respiratory diseases, such as cystic fibrosis, pneumonia, and bronchitis, thereby aiding in the clearing of respiratory airways and improvement of lung function.

Bovine DNase A, B, C, and D were purified and completely sequenced as early as 1973. Bovine pancreatic DNase has been sold under the trade name Dornavac (Merck), and was used in the treatment of patients subject to pneumonia and CF. This product was withdrawn from the market after clinicians observed serious complications from its use (Raskin, Am. Rev. Respir. Dis. 98:597-598 [1968]).

Human DNase I was cloned and expressed by Shak, *et al. ,* Supra who 35 reported that catalytic amounts of recombinant human DNase (rhDNase) greatly reduced the viscosity of purulent cystic fibrosis sputum. transforming it from a non-flowing viscous gel to a flowing liquid, thereby increasing sputum clearance. Hubbard *et al.,* Supra and Aiken *et al.,* JAMA 267:1947-1951 [1992]) reported improved lung function in CF patients upon treatment with aerosolized rhDNase.

### Effect of Mucus Rheology on Respiratory Airways

Respiratory secretions, such as mucus, play an important role in the normal functioning of the respiratory airways. These respiratory secretions protect the airways against airborne microorganisms and other foreign particles by providing a continuous flow, or transport, of secretions under the propelling action of ciliated epithelium. This transport helps in clearing the trapped particles and microorganisms in the mucus lining the airways. The viscoelastic gel properties of normal mucus are critical for effective mucociliary transport (Yeates *et al.,* Supra.

Patients subject to respiratory disease characterized by infected airway secretions have been shown to secrete excessive amounts of purulent secretions having abnormal viscoelastic properties. There is variability in the viscoelasticity of infected sputum dependent upon the respiratory disease type, severity, and duration that makes treatment regimes unpredictable.

King *et al.* (Rheology 24: 589-597 [1987]) show an inverse relationship between viscoelasticity and ciliary transport; the greater the viscoelasticity the less the ciliary transport. Impaired ciliary transport can lead to lung obstruction of airways by infected secretions that can cause respiratory distress, and in some cases, can lead to respiratory failure and death.

Researchers have attempted without success to find a correlation between in vitro and in vivo assessment of the rheologic properties of sputum. Reid *et al.,* ("Rheology-Relation to the Composition of Sputum" Scandinavian Journal of Respiratory Diseases Publisher: Munksgaard International Publishers Vol 103 Suppl. pg 27-35 [1974]) state that in vitro assessment of shear rates of sputum cannot be used to assess the rheological properties of sputum in vivo. Rosenbluth *et al.* (Archives of Disease in Childhood 49:606 [1974]) demonstrate that although increasing the water content of CF sputum in vitro reduces viscosity, mist therapy did not influence sputum viscosity or volume in patients with CF.

### Measurements of the Rheological Properties of Sputum

Relevant rheological measurements of the sputum of patients subject to respiratory disease can be difficult to obtain with a single rheological method. In fact, results from one technique are rarely comparable to results obtained by other techniques due to the inherent variability of the viscoelasticity of patient sputum samples and the non-Newtonian characteristics of sputum where the values of viscosity and elasticity depend on the method of analysis.

Several types of instruments have been used to study the rheological properties of mucus, including the dynamic cone and plate viscometer, the capillary rheometer, the oscillating magnetic ball micro-rheometer, and the magnetic rheometer. These instruments measure dynamic moduli related to viscosity and elasticity.

The dynamic cone and plate viscometer is one of the least destructive of these methods and can yield information simultaneously about both viscosity and elasticity of a specimen; however this method requires using relatively large volumes of sputum. The capillary rheometer, while requiring small sample volumes, has several limitations, including problems with sample handling. The magnetic micro-rheometer does not work satisfactorily for materials that are highly heterogenous such as sputum, because the particles' motion is often stopped when it moves into an area of higher viscosity.

Yeates *et al.* Supra, caution that comparisons of rheological values derived with different instruments need to be made with discretion, because each is dependent on the application of different rheological principles.

Many methods used to obtain rheological measurements are destructive, irrevocably altering the physical nature of the sputum during storage or data collection. This is true of freezing and the use of devices such as the static cone and plate rheometer, frequently used for viscosity determinations. Many instruments, including the dynamic cone and plate rheometer, are very expensive and require a trained technician to operate the instrument and to interpret the resulting data.

Until the present invention there was no simple, validated or standardized in vitro rheological assay to assess the potential of a therapeutic to improve lung function in a patient subject to respiratory disease. Until the present invention there was no simple, validated or standardized in vitro rheological assay to measure respiratory disease severity as a function of viscoelasticity. Until the present invention there was no method for assessing the therapeutic efficacy of rhDNase treatment on mammalian patients subject to respiratory disease.

US-A-3 914 985 discloses a centrifuge device and method for harvesting compacting and measuring particulate matter, especially cellular matter, in body fluids such as blood. Following centrifugation, the compacted particulate matter is subjected to volumetric measurement.

The compaction assay of the present invention is based upon the change in sputum compactability in a centrifugal field following in vitro DNase treatment of sputum. The extent of sputum compactability, as measured by centrifugal pellet size, is related to the content of large molecular weight DNA. The greater the amount of large molecular weight DNA in a sputum sample, the more resistance to compaction. DNase acts to hydrolyze the large molecular weight DNA present in purulent sputum thereby increasing the centrifugal compactability of an in vitro DNase treated sample. The greater the increase in compactability of an in vitro DNase treated sputum sample from a patient, the greater the improvement in patient lung function found with in vivo rhDNase treatment.

There exists a need for a simple and rapid standardized assay to assess the ability of a therapeutic to improve lung function in a population of patients. This need exists in any patient population subject to respiratory disease characterized by infected airway secretions.

In many instances, it would be desirable to assess the severity of respiratory disease in a mammalian patient in order to determine effective dosage regimens of therapeutics useful in the treatment of respiratory disease. It would be particularly desirable to have a rapid and simple in vitro assay predictive of in vivo efficacy of DNase therapy in a population of patients in need of DNase therapy.

Accordingly, it is an object of the present invention to provide a rapid and simple standardized assay that can assess the potential of a therapeutic to improve lung function in a population of patients.

It is an object of the present invention to provide a rapid and simple in vitro assay to assess the severity of respiratory disease in a mammalian patient in order to determine effective dosage regimens of therapeutics useful in the treatment of respiratory disease. It is a related object of the present invention to provide an in vitro assay that is predictive of in vivo efficacy of DNase therapy in a population of patients in need of DNase therapy.

These and other objects of the present invention will become apparent to those skilled in the art.

### Summary of Invention

The present invention is based on the unexpected experimental finding that in vitro measurements of viscoelasticity obtained by the compaction assay are predictive of the severity of respiratory disease in a mammalian patient and predictive of in vivo efficacy of therapeutics useful in the treatment of respiratory disease in improving lung function.

The objects of the present invention are accomplished by providing a method for measuring the compaction of a sputum sample from a mammalian patient subject to respiratory disease associated with infected airway secretions, comprising obtaining a sputum sample from the mammalian patient, centrifuging said sputum sample until fractionated into supernatant and pellet phases, and measuring the pellet.

In one embodiment, the sputum sample is from a mammalian patient subject to cystic fibrosis. In another embodiment, the sputum sample is from a mammalian patient subject to bronchitis.

In yet another embodiment, the present invention provides for a method of measuring the compaction of a mammalian patient sputum sample treated in vitro with a therapeutic, comprising obtaining a sputum sample from the mammalian patient, adding therapeutic to the sputum sample, centrifuging said sputum sample until fractionated into supernatant and pellet phases, and measuring the pellet.

In one embodiment, the therapeutic is human DNase. In another embodiment the therapeutic is an antibiotic useful in the treatment of respiratory disease.

In yet another embodiment of the present invention, a method is provided for measuring the compaction of a DNase treated sputum sample of a mammalian patient in need of DNase therapy comprising, obtaining a sputum sample from the mammalian patient, adding DNase to the sputum sample, centrifuging the DNase treated sputum sample until fractionated into supernatant and pellet phases, and measuring the pellet.

In another embodiment, the DNase is human DNase in a concentration of at least 1 µg DNase / mL sputum.

### Brief Description of the Figures

Figure 1 shows the nucleotide and amino acid sequences of DNase (SEQ. ID NO.1).

Figure 2 is an agarose gel electrophoresis study of DNA size within sputum samples. Sputum samples were prepared by treatment with either diluent, the minus lanes, or rhDNase, the plus lanes, and electrophoresed into a 0.5% agarose gel.

Figure 3 shows percent pellet heights for the six CF sputum samples obtained in the compaction assay following addition of either diluent, grey bars, or rhDNase, solid black bars.

Figure 4 shows viscosity and elasticity moduli measured as a function of strain by the dynamic cone and plate viscometer for a representative sputum sample.

Figure 5 shows the physical properties of six purulent samples.

Figure 6 shows the extent of DNA detected in the pellet and supernatant fractions of six samples following addition of diluent or rhDNase.

Figure 7 shows a dose-response curve for rhDNase action of a CF sputum sample. Purulent CF sputum was treated with increasing concentrations of rhDNase and evaluated by the compaction assay. Concentrations of rhDNase were calculated for the volume of sputum plus enzyme addition.

Figure 8 shows the relationship between compaction assay results and measured elasticity. Elasticity was measured by a dynamic cone and plate viscometer at 20% strain as described. The percent pellet (from compaction assay results) was correlated for the six samples characterized completely in this study as well as four other purulent CF sputum samples. Data points represent both diluent-treated (filled symbols) and rhDNase-treated (open symbols) samples.

### Description of the Preferred Embodiments

The use of the term "compaction" as used herein refers to the resistance to deformation of a sputum sample upon centrifugation and is a direct function of sample viscoelasticity. The more viscoelastic a sample, the less compactability the sample will have upon centrifugation as measured by centrifugal pellet size. Compaction assay results are useful in a clinical setting to assess respiratory disease severity, as a function of sputum viscoelasticity, and to assess the potential of therapeutics in improving lung function in mammalian patients subject to respiratory disease.

The therapeutic efficacy of DNase in improving lung function in a mammalian patient in need of DNase therapy can be predicted by assaying compactability of the mammalian patient sputum sample following in vitro treatment with DNase. If centrifugal pellet size decreases upon in vitro DNase treatment, it is predicted that treatment of the mammalian patient with human DNase will improve lung function. The reduction of pellet size after DNase treatment is due to the hydrolysis of long chain DNA to shorter chains. The subsequent release of the short chain DNA into the supernatant results in increased pellet compaction and increased supernatant volume. This effect can be quantified and used as an indicator of the extent of viscoelasticity change after in vivo rhDNase treatment. Those patients in need of DNase treatment are predicted to be responsive to DNase therapy if in vitro rhDNase treatment increases compaction of the patient's sputum sample.

Centrifugation of an untreated sputum sample from a mammalian patient in need of DNase treatment yields a sample having a DNA concentration greater in the centrifugal pellet than in the remaining supernatant. Because of the dehydrated condition of the sputum of mammalian patients in need of DNase therapy, centrifugation at 10,000Xg alone does not always produce a supernatant phase. It is often necessary to add diluent to the sputum sample in order to obtain centrifugal fractionation of the sample into two phases and the amount of diluent added should be the same for all samples being tested. If a positive displacement pipette is used for measuring the sputum sample to be tested, it is preferable to add diluent to the sputum sample in a volume equaling 50% of the measured sample volume to insure at least a 20% supernatant volume upon centrifugation. If the sputum sample is measured gravimetrically it is preferable to add diluent to the sputum sample in a volume equaling 50% of the exact sputum weight to yield at least a 20% supernatant volume upon centrifugation. For example, if the exact sputum volume as measured by a positive displacement pipette is 100 microliters (µl), then addition of 50 µl of diluent is typically necessary to yield at least a 20% supernatant volume upon centrifugation or for example, if the exact sputum sample weight is 100 milligrams (mg), then addition of 50 µl of diluent is typically necessary to insure at least a 20% supernatant volume upon centrifugation. Typically, a Mettler analytical balance is used for measuring sputum sample weight. Unless a positive displacement pipette is used for measuring the sputum sample volume, the addition of diluent volume based on sputum sample weight is necessary due to the presence of air bubbles and mucus plugs in the sputum sample which can affect volume measurements. After the addition of diluent or DNase and subsequent centrifugation, the resulting supernatant is neither elastic nor viscous, however, the remaining pellet is quite solid or elastic. The remaining pellet phase contains the components that contribute to the elasticity of whole sputum.

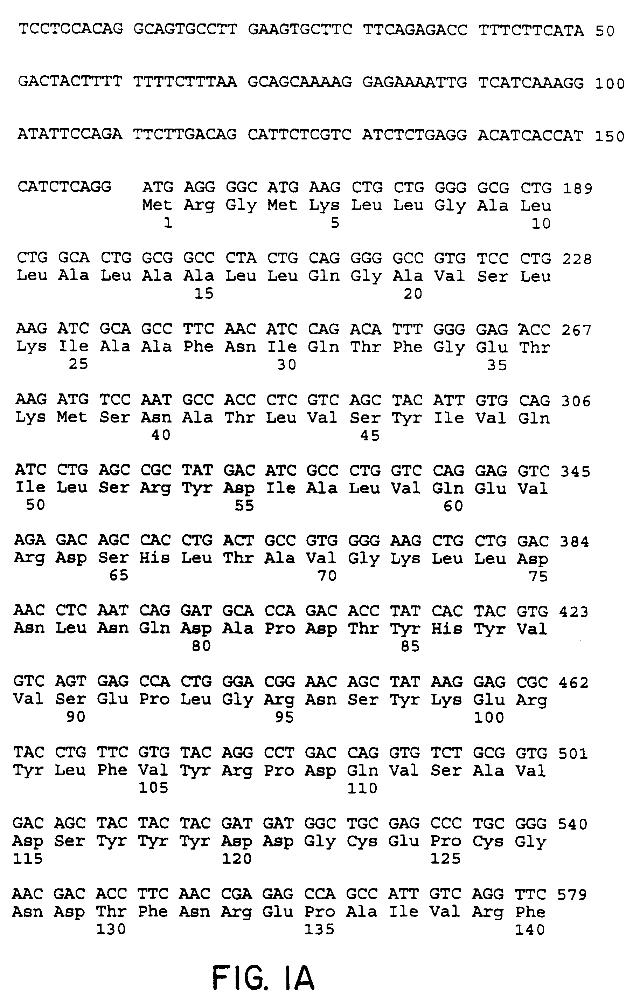

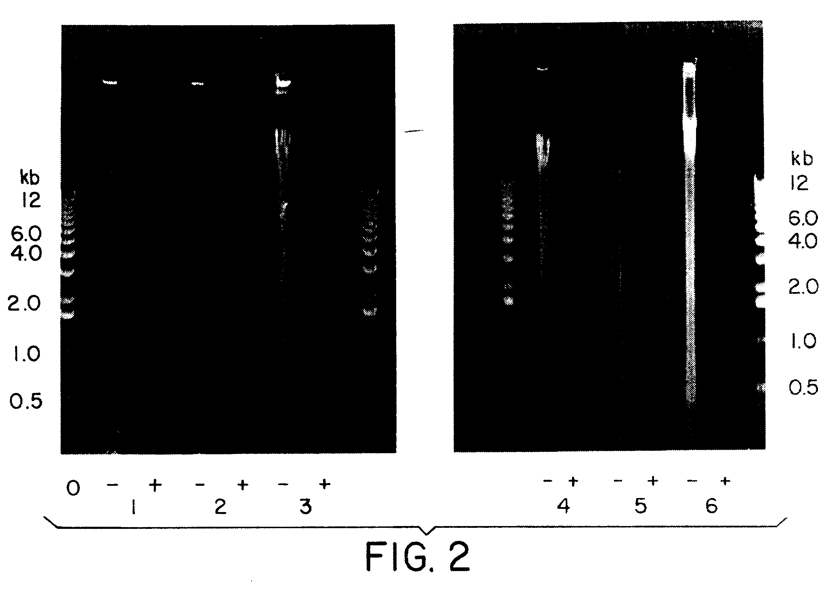

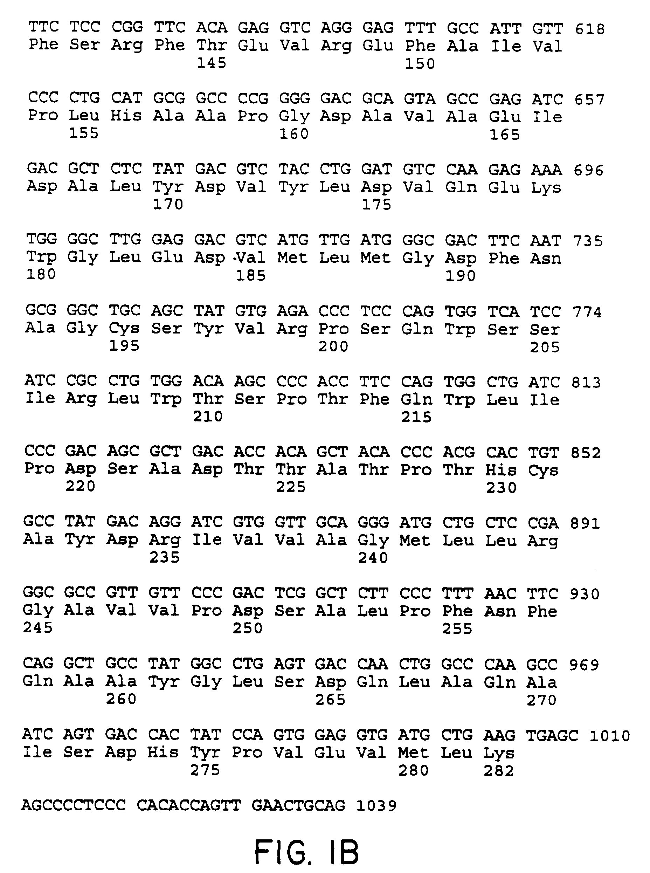

DNase is defined as a polypeptide having the amino acid sequence of Figure 1, together with amino acid sequence variants thereof which retain the qualitative enzymatic activity of DNase. As used herein the term "DNase" refers variously to all forms of human and non-human animal DNase as are known to be biologically active in accepted DNase assays, such as ELISA, RIA, hydrolysis of ³²P-labeled DNA, or PAGE electrophoresis, as described in WO90/07572, published 12 July 1990 or agar plate DNase assay (Smith *et al.,* Applied Microbiology 18:991[1969]) and is meant to include DNase in a mature, pro, or met form, whether obtained from natural source, chemically synthesized or produced by techniques of recombinant DNA technology.

A complete description of the preparation of recombinant human DNase (rhDNase) including its cDNA and amino acid sequences and expression is shown in Shak *et al.,* PNAS 87:9 188-9192 [1990]) and W090/07572. Non-human animal DNase, including bovine, porcine, and ovine are, for example, disclosed in Liao *et al.* (J. Biol. Chem. 248:1489 [1973]); Salnikow *et al.* (J. Biol. Chem. 248:1499 [1973]); Liao *et al.* J. Biol. Chem. 249:2354 [1973]); Paudel *et al.* (J. Biol. Chem. 261:16006 [1986]); and Paudel *et al.* (J. Biol. Chem. 261:16012[1986]).

The term DNase includes variously glycosylated forms and other variants and derivatives of such DNases, whether known in the art or becoming available in the future. Examples of such variants are alleles, and the products of site directed mutagenesis in which residues are deleted, inserted and/or substituted. Preferably, the variants are not immunogenic in humans. Variants may possess greater enzymatic activity, improved solubility, or may be expressed at higher levels by host cells.

DNA encoding human DNase is synthesized by in vitro methods or is obtained readily from human pancreatic cDNA libraries as described in Shak *et al.,* Supra. Expression of rhDNase is described in Shak *et al.,* Supra and WO90/07572.

Human DNase is placed into therapeutic formulations together with required cofactors, and optionally is administered in the same fashion as has been the case for animal DNase such as bovine pancreatic DNase. The preferred formulation for human DNase is a buffered or unbuffered solution, and is preferably an isotonic salt solution such as 150 mM sodium chloride, containing 1.0 mM calcium at pH 7. The concentration of sodium chloride may range from 75mM to 250 mM. The concentration of calcium may range from 0.01 to 0.05 mM, and other divalent cations that stabilize DNase may be included or substituted for calcium. The pH may range from 5.5 - 9.0, and buffers compatible with the included divalent cation may also be utilized. These solutions are particularly adaptable for use in commercially-available nebulizers including jet nebulizers and ultrasonic nebulizers useful for administration, for example directly into the airways or lungs of a patient subject to respiratory disease associated with infected airway secretions. The formulation may be lyophilized powder, also containing calcium.

Commercially available nebulizers for liquid formulations, including jet nebulizers and ultrasonic nebulizers are useful for administration. Liquid formulations may be directly nebulized and lyophilized powder nebulized after reconstruction. Alternatively, DNase may be aerosolized using a fluorocarbon formulation and a metered dose inhaler, or inhaled as a lyophilized and milled powder.

Purified DNase is employed for enzymatic alteration of the viscoelasticity of sputum. Human DNase is particularly useful for the treatment of patients with pulmonary disease who have abnormal, viscous or inspissated purulent secretions in conditions such as acute or chronic bronchopulmonary disease (infectious pneumonia, bronchitis or tracheobronchitis, bronchiectrasis, cystic fibrosis, asthma, TB or fungal infections), atelectasis due to tracheal or bronchial impaction, and complications of tracheostomy. For such therapies a solution or finely divided dry preparation of human DNase is instilled in conventional fashion into the bronchi, e.g. by aerosolization of a solution of DNase.

Chemical action of in vitro rhDNase treatment of sputum samples can be verified by using agarose gel electrophoresis following the procedure of Shak *et al.,* Supra and WO90/07572.

DNase may also be administered along with other pharmacologic agents used to treat the conditions listed above such as antibiotics, bronchodilators, anti-inflammatory agents, and mucolytics e.g. n-acetylcysteine.

Mammalian patients in need of DNase therapy are those patients subject to respiratory disease characterized by infected airway secretions.

The therapeutically effective amount of DNase used in the treatment of human patients subject to respiratory disease associated with infected airway secretions is a dosage of from about 1 µg to about 100 mg of human DNase per kilogram of body weight of the patient, administered with pharmaceutical compositions, as described herein. The therapeutically effective amount of human DNase will depend, for example upon the therapeutic objectives, the route of administration, and the condition of the patient. Accordingly, it will be necessary for the therapist to titer the dosage and modify the route of administration as required to obtain the optimal therapeutic effect.

### DNA in sputum of patients subject to respiratory disease

In a number of respiratory diseases the deoxyribonucleic acid (DNA) content of sputum increases with the degree of purulence (Picot *et al.,* Thorax 33:235-242 [1978]). This is particularly the case in CF where high levels of infection are associated with increased levels of sputum DNA (Carswell *et al.,* Eur. J. Respir Dis 65: 53-57 [1984]). There is inherent variability in the properties of DNA obtained from the sputum samples of patients subject to respiratory disease.

DNA influences the viscoelasticity of sputum through the interaction with other components of sputum including the mucus glycoproteins.

The DNA present in the sputum of patients subject to respiratory disease originates from either infecting organisms or from the patient's own cells (Lethem, *et al.,* Eur. Respir J., 3:19-23 [1990]). If the host cells are the major contributor to total sputum DNA, this is a reflection of the content of leukocytes and epithelial cells present due to inflammation. Lethem, *et al.* Supra report that the DNA co-purifying with mucus glycoproteins is human in origin which suggests that it is the inflammatory processes resulting from infection, rather than the infection itself which are the source of the DNA in CF sputum.

### Use of Antibiotics in Respiratory Disease

The presence of purulent respiratory secretions in the airways of patients subject to respiratory disease makes an excellent growth medium for microorganisms, and pulmonary infections are commonplace despite normal host defense mechanisms. The use of antibiotics is indicated for these infections.

The three most common bacterial pathogens isolated from the sputum of cystic fibrosis (CF) patients are *Staphylococcus aureus, Pseudomonas aeruginosa,* and *Hemophilus influenzae. Proteus* and *Klebsiela* species are observed much less frequently. The presence of these bacteria is believed to be responsible for some of the destructive changes in the lungs of cystic fibrosis patients.

Chronic sinusitis is also found in CF and may result from obstruction of the sinus ducts which prevents drainage and is not related to the underlying lung disease. The bacteria generally isolated in these cases of sinusitis include *P. aeruginosa, H. influenzae, streptococci,* and *anaerobes.*

Although the use of antibiotics in CF is both controversial and fraught with difficulty (In Pharmacotherapy: a Pathophysiologic Approach Editors: DiPiro and Talbert *et al.,* Publisher Elsevier New York, pg 836-843 [1989]), most clinicians do treat pulmonary infections associated with CF with antibiotics. Specific therapy is directed at proven or likely pathogens such as *Pseudomonas aeruginosa* and *Staphylococcus aureus,* and usually includes an aminoglycoside and an extended-spectrum penicillin. While complete eradication of the pathogen is a practical endpoint in antibiotic therapy, the total eradication of *Pseudomonas aeruginosa* is infrequent and transient. Therefor a practical endpoint for the use of antibiotics is a return to the preexacerbation clinical status or pulmonary function status.

Another problem with antibiotic use in CF is altered pharmacokinetic disposition of some antibiotics in most CF patients. Many CF patients have increased total body clearance for many antibiotics and therefore higher doses of these agents may be necessary to produce therapeutic concentrations. Unfortunately, these alterations in pharmacokinetics are not consistent or predictable. Upward adjustments in dosage should be made with some degree of caution and should be followed with further determination of serum concentrations.

Although controversy exists surrounding the use of antibiotics in chronic bronchitis (In Pharmacotherapy: a Pathophysiologic Approach Editors: DiPiro and Talbert *et al.,* Publisher Elsevier New York, pg. 1092-1095 [1989]), the antibiotics most frequently selected in the treatment of chronic bronchitis (ampicillin, tetracycline, chloramphenicol, trimethoprimsulfamethoxazole) possess in vitro activity against the common sputum isolates H. *influenzae, S. pneumoniae,* and M. *pneumoniae*.

Numerous antibiotics are available for the treatment of pneumonia. Once a causative pathogen(s) has been identified, an antibiotic regimen can be targeted to the particular pathogen(s) identified. Antibiotics useful in the treatment of pneumonia include but are not limited to erythromycin, ampicillin, penicillin, semi-synthetic penicillin, tetracycline, cefuroxime, cephalosporin, clindamycin, aminoglycoside, and ticarcillin. A complete listing of antimicrobial agents for specifically directed therapy can be found in The Medical Letter Handbook of Antimicrobial Therapy (In Pharmacotherapy: a Pathophysiologic Approach Editors: DiPiro and Talbert *et al.,* Publisher Elsevier New York, pg. 1100-1105 [1989]).

The term "rheology" and grammatical derivatives thereof as used herein refer to the deformation and flow properties of a substance. With a viscoelastic substance such as sputum the flow properties are based on viscosity and elasticity.

Several types of rheometers have been used to study the rheological properties of mucus. Rotational rheometers have either a coaxial-cylinder (Davis "Rheological Examination of Sputum and Saliva and the Effects of Drugs, In: Gabelnick HL, Litt M, eds. Rheology of Biologcal Systems. Springfield, II: Charles C Thomas pub pg. 158-193 [1973]), a cone and plate (Mitchell-Heggs "Physical Properties of Microliter Quantities of Normal Mucus" In: Elstein and Parke Eds. Mucus in Health and Disease, Advances in Experimental Medicine and Biology vol 89. New York: Plenum Press, pg. 203-215 [1977]), or a parallel-disk sensor system (Puchelle Biorheology 21:265-272[1984]). These rheometers can be used at a constant speed of rotation, or they can be used in an oscillatory mode. These rheometers necessitate the use of shear strains that are 200-1000 times those estimated for cilia. These high shear rates are almost impossible to correlate with in vivo conditions.

In the cone and plate rheometer, the fluid under test is placed between the cone and plate. One of these two platens is rotated which applies a shearing force known as the shear rate. The more viscous the liquid the more stress is set up within it, and is conducted to the upper platens and measured as shear stress. Viscosity is then calculated as the ratio of shear stress to shear rate. Percent strain represents the amplitude of oscillation in units of the spacing between the cone and plate. Thus the lower the percent strain, the lower the oscillation amplitude and the less damage done to the sputum sample.

Magnetic rheometers have been developed using micron-sized ferromagnetic particles that can induce small strain, 10-50 times those estimated for cilia. Although there is better in vivo correlation of data with the magnetic rheometer than with the rotational rheometer, the magnetic rheometer is experimentally complex and not suitable for routine testing. Also, mathematical analysis of data from the magnetic rheometer requires the use of a digital computer, which is not suitable for routine clinical use.

Capillary rheometers have been used to measure physiologic fluid viscosity and elasticity for small samples. Viscosity is measured by determining the fluid flow rate in response to a known pressure drop. The recoverable shear strain is determined when this differential pressure is returned to zero. The strains applied to induce fluid flow in capillaries in these instruments are about 10,000 times those estimated to be produced by the cilia and therefore do not correlate with in vivo conditions (Yeates *et al.,* Supra.

### Rheologic properties of sputum

Mechanical clearance of the airways occurs by way of the mucociliary transport system whose functioning mainly depends on the interaction of two major elements: the cilia which correspond to the motor element of the system and the mucus which is the transport element. Secretions are propelled along the lining membranes of respiratory air passages by the activity of the cilia. The mucus is made up of a 2-layer system: the lower non viscid layer, also termed periciliary, and the upper gel viscoelastic layer. Puchelle (Biorheology 21:265-272 [19841) reports that the mucociliary transport rate will decrease with increased viscosity of the periciliary layer . The increased viscosity and elasticity of respiratory mucus in patients subject to respiratory disease characterized by infectious secretions of the airways makes it difficult for them to clear the secretions from the airways, thus contributing to pulmonary insufficiency, lung infection, and death.

Viscosity, the ability of a material to resist deformation, is the most significant property affecting the flow behavior of a fluid. Viscosity is that property of a fluid or semi-fluid that enables it to develop and maintain an amount of shearing stress dependent upon the velocity of flow and then to offer continued resistance to flow.

Newton's law of viscosity states that this force per unit area is proportional to the velocity gradient between the layers. Thus, τ = µy, where τ = shear stress, µ = viscosity, and *y* = shear rate. Fluids that follow this behavior are described as Newtonian fluids.

Non-Newtonian fluids do not obey Newton's law of viscosity, thus the viscosity is dependent on the shear rate. Viscosity decreases with increasing shear. Shear is movement of a layer relative to that of an adjacent layer. Sputum is an example of a non-Newtonian fluid. When a shear stress is applied to sputum, it will behave as a gel and deform roughly in proportion to the stress applied. The shear strain is the ratio of the distance the surface moves compared to the depth of the sputum layer and thus is dimensioniess.

Elasticity refers to the ability of sputum to store energy, or resist deformation, when subjected to shear forces. Those fluids that are markedly deformed with very little applied stress but that still return to their original shape are referred to as highly elastic. Sputum samples before rhDNase treatment exhibit greater elasticity than viscosity, although the differences are substantially less after rhDNase treatment. Elasticity is more greatly affected by rhDNase treatment than is viscosity, although both decrease. Elasticity is not dependent on the total DNA concentration. A relationship does exist however between the amount of high molecular weight DNA and the measured elasticity.

The property of viscoelasticity refers to the conjoint properties of viscosity and elasticity exhibited by physiologic fluids including sputum. Lethem *et al.* (Eur Respir J. 3:19-23 [1990]) have demonstrated CF sputum viscoelasticity to increase following exogenous DNA addition and Picot *et al.,* Supra show the viscoelasticity of sputum was found to be independent of total DNA content but increased greatly with the in vitro addition of unhydrolyzed long chain calf thymus DNA.

Percent dry weight of sputum is a measurement of the percentage of solids that exists after drying the sputum sample 2 hours at 160° C. Percent dry weight was calculated as 100 minus the % weight lost after drying.

The term "sputum" as used herein refers to expectorated matter made up of saliva and discharges from the respiratory airways. Sputum is a non-Newtonian highly complex material that has a pronounced gel-like structure. Sputum that is purulent has increased viscoelasticity attributed to both mucus glycoproteins and DNA and is characterized by the fluid products of inflammation for example, leukocytes and the debris of dead cells. There is inherent variability in the rheological properties of purulent sputum.

For collection of sputum for use in the assay of the present invention, Byrne, *et al.,* (Laboratory Tests: Implications for Nursing Care 2nd Edition Editors Byrne *et al.,* Publisher Addison-Wesley California [1986]) suggest that the patient collect material, raised by several deep coughs, in a container with a lid. Alternatively, sputum can be collected by using a bronchoscope as described in Kim. *et al.,* (Bull. Europ. Physiopath Resp. 18:915-927 [1982]).

### Centrifugation

Centrifuges are used to isolate solids from liquids or one liquid from another. Centrifuges are also used to effect the settling of a light phase from a heavy phase or to filter suspended solids by magnifying the force of gravity.

Centrifuges are designed to utilize the principle that an object spinning about a central point at a fixed radial distance is acted upon by a force. Although the velocity of the object is constant, its direction is constantly changing and it is acted upon by a centripetal force toward the center of rotation.

Centrifuges generally consist of a rotor or bowl in which the centrifugal force is enacted upon its components, a system to drive and spin the bowl about its axis, a frame to support the system, and an enclosure to contain the rotor and keep the separated products in a discrete state.

In industrial centrifuges, the centrifugal acceleration is many times the gravitational acceleration. Centrifugal force varies with rotational speed and with radial distance from the center of rotation.

### Dose of rhDNase

In the present invention, the minimum in vitro rhDNase concentration showing activity in the compaction assay was 1 µg rhDNase/mL sputum. Using one of the current rhDNase dosage forms, 0.25 mg /mL, and assuming that 0.5 mL of the formulation is actually delivered to the lungs and that the sputum volume over which the concentration of rhDNase is deposited is 100 mL, it can be estimated that the final concentration of rhDNase in vivo is 1.25 µg / mL.

Clinical studies using 1 and 4 mg/mL rhDNase solutions have resulted in increased pulmonary function in CF patients (Aitken *et al.,* Supra and Hubbard *et al.,* Supra). After inhalation of 10 mg of rhDNase, concentrations of rhDNase in sputum collected one hour after inhalation typically ranged from 1-20 µg / mL. This is in agreement with the in vitro data and suggests that the concentration of rhDNase in the formulation is sufficient to have an effect on reducing sputum viscoelasticity. Concentrations of rhDNase above the minimum threshold are preferred in order to allow for variations of sputum characteristics and aerosol deposition.

The use of the term "therapeutic" as used herein refers to those agents effective in the treatment of respiratory disease associated with infected airway secretions and includes but is not limited to antibiotics, DNase, mucolytics. antineutropnil elastase agents, and secretory leucoprotease inhibitor.

Further details of the invention are illustrated in the following non-limiting example.

### Example 1

### Compaction Assay of the Sputum of CF Patients Treated with rhDNase

This example shows that in vitro DNase treatment of purulent sputum from patients subject to respiratory disease characterized by infected airway secretion hydrolyzes the long chain DNA present in the samples thereby reducing the viscosity of the sputum. This example shows that results from the compaction assay of the present invention correlate with rheological measurements obtained from the plate and cone rheometer.

### Specimen Characterizations and Test Solutions

Purulent sputum samples obtained from hospitalized, rhDNase-naive Cystic Fibrosis (CF) patients were divided into aliquants for DNA evaluation and rheological analysis. The aliquants for total DNA content and length were frozen until analysis and those for rheological measurements were refrigerated until tested (within 24 hours of collection). Sputum dry weight was determined gravimetrically after drying for 2 hours at 160° C. Percent dry weight was calculated as 100 minus the % weight lost after drying. The gene for DNase I was cloned and expressed at Genentech, Inc. as described in Shak *et al.* Supra [1990]. The expressed rhDNase was used as a 4 mg/ml solution in 1mM CaCl² and 150 mM NaCI at pH 7.4. Control additions, termed "diluent", and dilutions of 4 mg/ml rhDNase used the same salt solution minus rhDNase.

### Total DNA Content and Length Assays

Sputum sample DNA content was determined by a modification of the diaminobenzoic acid (DABA) assay developed by Kissane and Robins (J Biol Chem 233:184-188 [1958]) which quantitates total DNA concentration independent of length. Previously frozen sputum aliquants were diluted ten fold with the assay diluent (25 mM HEPES, 1 mg/ml bovine serum albumin, 4 mM CaCl₂, 4 mM MgCl₂ 0.05% polysorbate 20, and 0.01% thimerosol, at pH 7.5) and incubated at 60° C for one hour. Diluted specimens (50 µLl were pipetted into microtiter plate wells and 50 µL of a 20% 3,5-diaminobenzoic acid hydrochloride (DABA) solution was added after which the plates were tightly sealed. After one hour of incubation at 60° C the reaction was stopped by the addition of 50 µL of 5M HCl. A Cytofluor (Millipore Corp.) microtiter plate fluorometer (with 390 nm excitation and 530 nm emission filters) was used to read the plates. Salmon testes DNA (Sigma) was used to establish a standard curve. Sputum DNA concentrations from 0.125 to 8 mg/ml were measurable. The coefficient of variation for the assay was determined to be ≤6%.

The length of the DNA in sputum sampies was determined qualitatively by agarose gel electrophoresis as described by Shak *et al.*, Supra. The effect of freezing on sputum DNA length was studied by electrophoresing samples with and without one cycle of freezing at -70 °C. Samples were diluted four-fold in a buffer containing 100 mM NaCI, 5 mM EDTA, 0.5% SDS, 100 µg/ml proteinase K (Sigma), 50 mM Tris, pH 8.0, and incubated for three hours at 37 °C with occasional mixing. Sampies were then electrophoresed in 0.5% agarose gels and stained with ethidium bromide by standard procedures (Sambrook, *et al.,* Molecular Cloning: A Laboratory Manual, 2nd ed. Cold Spring Harbor Laboratory Press [1989]). A 1 Kb DNA ladder having 12 major bands between 500 bp to 12 Kb (Gibco/BRL 5615SA) was used as the molecular weight standard.

### Rheometry

Sputum rheological measurements were made at ambient temperature using the Rheometrics Fluid Rheometer (RFR-7800, Rheometrics Inc. Piscataway, NJ) with a 0.02 radian, 25 mm radius cone and plate tool. Approximately 0.8 ml sputum was placed on the plate with 25 µL of diluent or rhDNase layered onto the top of the sputum. The cone was lowered onto the sample while the bottom plate was slowly rotated at 5 rpm to evenly distribute the sample across the gap between the cone and plate. Drying at the sample edge was prevented by pipetting 0.1 poise silicon oil (Dow Chemical) onto the exposed edge of the cone. Rotation of the plate was stopped for five minutes to allow the sample to relax. Each preparation was tested at a deformation rate of 10 radians/sec and the percent strain was varied from 10% to 200% in 10% increments. Percent strain is defined as 100 times the distance of the shearing motion divided by the space between the cone and plate of the viscometer. The second strain value, 20% strain, was used for the comparisons between samples and treatments, being the lowest possible strain for accurate measurement. Instrument start-up frequently compromises the first data point and higher strains can damage network structures (Ferry, J Viscoelastic Properties of Polymers. 3rd ed. John Wiley and Sons, Inc. New York pg. 245- [1980]) resulting in artifacts and irreproducible data.

### Compaction assay

Approximately 100 µL of sputum was transferred into a tared Eppendorf microcentrifuge tube with a positive displacement pipet (Gilson Microman®, equipped with a stainless steel shaft and plastic plunger). Either rhDNase or control diluent was added as volume equaling 50% of the exact sputum sample weight. Eppendorf tubes were vigorously vortexed for 15 seconds and then incubated for 15 minutes at ambient temperature. Aliquants of the treated sputum were loaded into Kimax-51, 1.5-1.8 x 90 mm melting point capillary tubes (Kimble Products) using 50 µL Drummond glass capillary tube (Microcaps). The loaded tubes were centrifuged for 20 minutes at 12,000 rpm in a Biofuge A microcentrifuge (Heraeus Sepatech GmbH) equipped with a horizontal, hematocrit-type rotor head (Heraeus Sepatech) and the supernatant/pellet interface subsequently inspected for resolution. On occasion some samples required up to 20 additional minutes to resolve the supernatant-pellet interface clearly. In these cases, all samples loaded on the rotor were centrifuged for the greater duration. The total height of the material loaded into the capillary tubes and the pellet height were measured in millimeters. The percent pellet was calculated as the height of the pellet divided by the height of the total material loaded, multiplied by 100.

### Results:

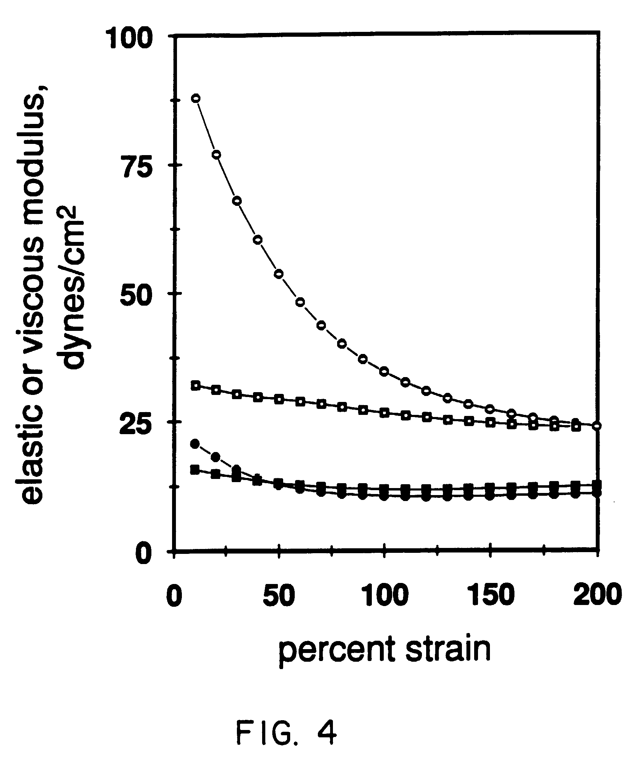

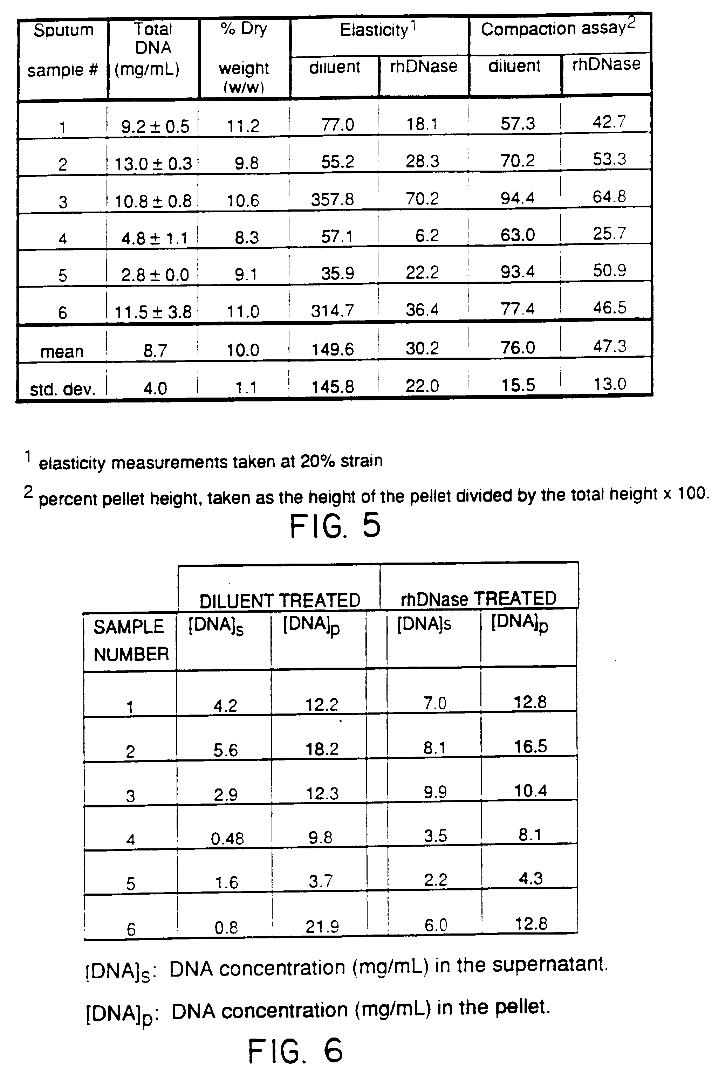

Viscosity and elasticity moduli measured as a function of strain by the dynamic cone and plate viscometer for a representative sputum sample are shown in Figure 4. Although treatment with rhDNase reduced measured values of viscosity over the range of strain tested, elasticity was more greatly affected. Using elasticity as the more dramatic rheological criterion for rhDNase effects of CF sputum, several physical properties of six purulent samples were evaluated and presented in Figure 5. Total DNA concentrations, average percent dry weights, responsiveness to rhDNase treatment assessed by dynamic cone and plate rheometry and the compaction assay showed all six samples to have a considerable DNA content but variable responsiveness to rhDNase treatment. In agreement with previous studies, Rosenbluth *et al.,* Supra the percent dry weight of the sputum sample does not correlate with either initial elasticity or initial viscosity.

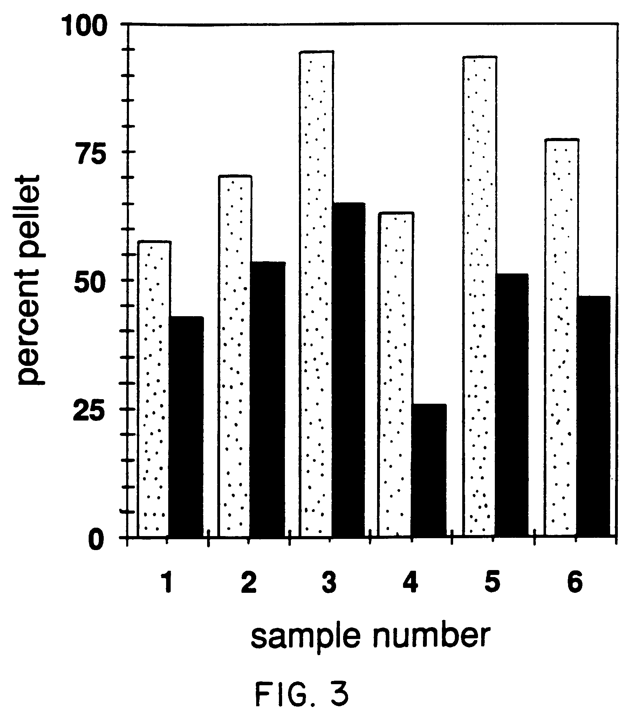

The agarose gel shown in Figure 2 demonstrates that prior to rhDNase treatment all six samples had variable amounts of very large DNA. Some DNA was too large to enter the gel and was retained at the application site. Smaller fragments of DNA, (varying from approximately 500 base pairs to greater than 12 Kb) could be resolved. All six sputum samples demonstrated complete hydrolysis (less than 500 base pairs) following incubation with 36 mcM rhDNase for 20 minutes at 25° C.

Total DNA was detected to a much greater extent in the pellet fraction than in the supernatant of these rhDNase-naive samples following diluent addition and centrifugation in the compaction assay, as demonstrated by Figure 6. The ratio of DNA distribution between the pellet and supernatant of these rhDNase-naive samples demonstrated a wide range. Samples 4 and 6 had the greatest fraction in the pellet and this correlated with the agarose gel electrophoresis study (Figure 2) which suggested these same two samples to have the greatest percent of large molecular weight DNA. Sample 3 had the next highest pellet/supernatant DNA ratio (Figure 6) and qualitatively, the next highest amount of large DNA (Figure 2). Samples 1,2, and 5 had the lowest pellet/supernatant DNA ratios and DNA of obviously smaller size.

Following rhDNase treatment. all six samples showed a redistribution of total DNA from the pellet to the supernatant following compaction (Figure 6). This observation coincides with tne agarose gel data (Figure 21, demonstrating a dramatic reduction in DNA size for each of these samples.

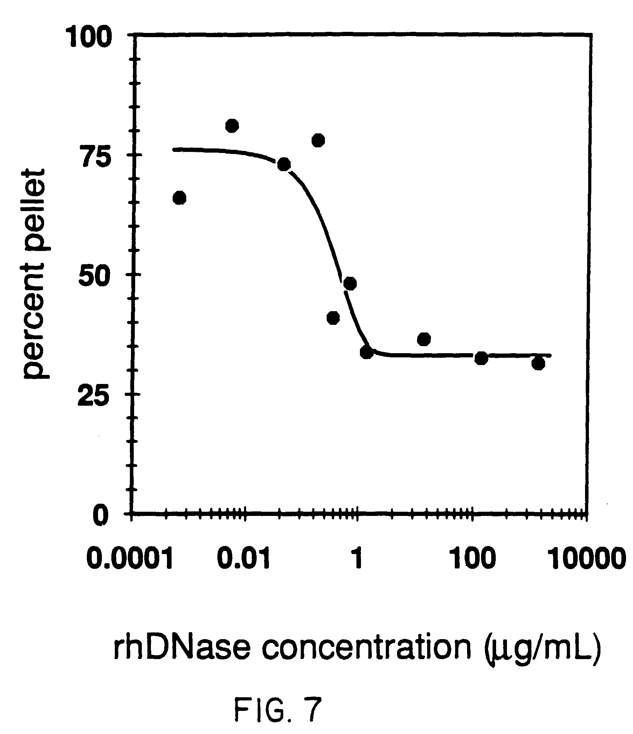

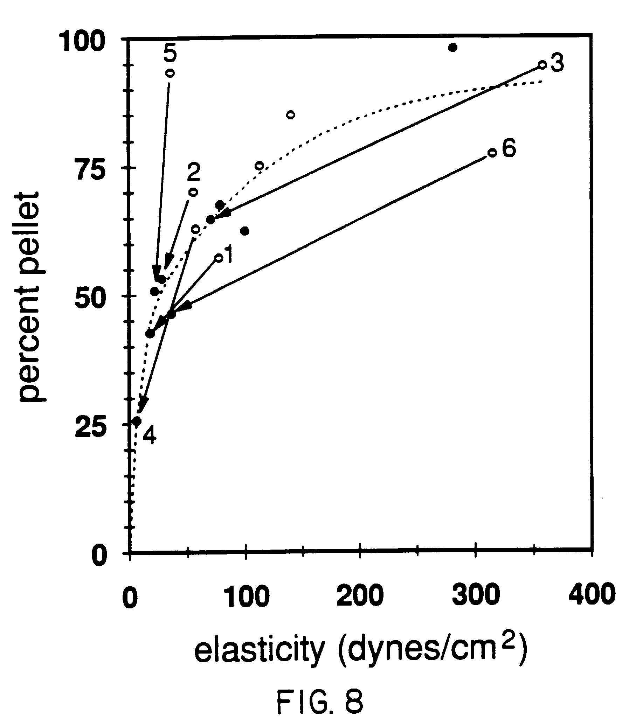

The percent pellet heights for the six CF sputum samples obtained in the compaction assay following addition of either diluent of rhDNase are shown in Figure 3. This data (as well as data from other CF sputum samples can be correlated to elasticity measurements obtained by the dynamic cone and plate rheometer (Figure 8). Together these results suggest that the compaction assay is a useful indicator of rhDNase action on purulent CF sputum. The compaction assay was used to obtain a dose-response curve for rhDNase action on a CF sputum sample (Figure 7). Fitting this data suggests rhDNase to be minimally effective below 0.1 µg/ml and maximally effective above 1 µg/ml in the CF sputum.

Although the foregoing refers to particular preferred embodiments, it will be understood that various modifications may be made.

## Claims

1. A method for measuring the compaction of a sputum sample from a mammalian patient subject to respiratory disease associated with infected airway secretions, comprising obtaining a sputum sample from the mammalian patient, centrifuging said sputum sample until fractionated into supernatant and pellet phases, and measuring the pellet.

2. The method of claim 1 wherein diluent is added to the sputum sample prior to centrifugation.

3. The method of claim 2 wherein the diluent is water added in a volume equaling 50% of the sputum weight.

4. The method of claim 1 wherein said mammalian patient sputum is from a patient subject to cystic fibrosis.

5. The method of claim 1 wherein said mammalian patient sputum is from a patient subject to pneumonia.

6. The method of claim 1 wherein said mammalian patient sputum is from a patient subject to bronchitis.

7. The method of claim 1 wherein said mammalian patient sputum is from a patient subject to sinus infections.

8. The method of claim 1 wherein said mammalian patient has been treated with a therapeutic.

9. The method of claim 1 wherein said mammalian patient has been treated with DNase.

10. The method of claim 1 wherein said mammalian patient has been treated with chest physiotherapy.

11. A method for measuring the compaction of a mammalian patient sputum sample treated in vitro with a therapeutic, comprising obtaining a sputum sample from the mammalian patient, adding therapeutic to the sputum sample, centrifuging said sputum sample until fractionated into supernatant and pellet phases, and measuring the pellet.

12. The method of claim 11 wherein said mammalian patient sputum is from a patient subject to respiratory disease associated with infected airway secretions.

13. The method of claim 11 wherein said respiratory disease is cystic fibrosis.

14. The method of claim 11 wherein said respiratory disease is bronchitis.

15. The method of claim 11 wherein said respiratory disease is pneumonia.

16. The method of claim 11 wherein said therapeutic is rhDNase.

17. The method of claim 11 wherein said therapeutic is an antibiotic useful in the treatment of respiratory disease.

18. The method of claim 11 wherein the therapeutic is added in a volume equaling 50% of the of the sputum sample weight.

19. A method of assaying the compaction of a DNase treated sputum sample from a mammalian patient in need of DNase therapy comprising, obtaining a sputum sample from the mammalian patient, adding DNase to the sputum sample, centrifuging the DNase treated sputum until fractionated into supernatant and pellet phases, and measuring the pellet.

20. The method of claim 19 wherein said mammalian patient is human and said DNase is recombinant human DNase I (rhDNase I).

21. The method of claim 19 wherein said recombinant human DNase I is in a concentration of at least 1 µg rhDNase I / mL sputum.

22. The method of claim 19 wherein said DNase is added in a volume equaling 50% of the sputum weight.

## Patentansprüche

1. Verfahren zur Messung der Kompaktion einer Sputumprobe von einem Säugetierpatienten, der an einer Erkrankung der Atmungsorgane leidet, die mit infizierten Atemwegssekreten einhergeht, umfassend das Abnehmen einer Sputumprobe vom Säugetierpatienten, das Zentrifugieren der Sputumprobe, bis sie in eine Überstand- und eine Pelletphase fraktioniert ist, und das Messen des Pellets.

2. Verfahren nach Anspruch 1, worin der Sputumprobe vor dem Zentrifugieren Verdünnungsmittel zugegeben wird.

3. Verfahren nach Anspruch 2, worin das Verdünnungsmittel Wasser ist, das in einem Volumen, das 50% des Gewichts des Sputums entspricht, zugegeben wird.

4. Verfahren nach Anspruch 1, worin das Sputum des Säugetierpatienten von einem Patienten mit cystischer Fibrose stammt.

5. Verfahren nach Anspruch 1, worin das Sputum des Säugetierpatienten von einem Patienten mit Lungenentzündung stammt.

6. Verfahren nach Anspruch 1, worin das Sputum des Säugetierpatienten von einem Patienten mit Bronchitis stammt.

7. Verfahren nach Anspruch 1, worin das Sputum des Säugetierpatienten von einem Patienten mit Nebenhöhleninfektion stammt.

8. Verfahren nach Anspruch 1, worin der Säugetierpatient mit einem Therapeutikum behandelt worden ist.

9. Verfahren nach Anspruch 1, worin der Säugetierpatient mit DNase behandelt worden ist.

10. Verfahren nach Anspruch 1, vorin der Säugetierpatient mit Brustphysiotherapie behandelt worden ist.

11. Verfahren zur Messung der Kompaktion einer in vitro mit einem Therapeutikum behandelten Säugetierpatienten-Sputumprobe, umfassend das Abnehmen einer Sputumprobe vom Säugetierpatienten, das Zugeben von Therapeutikum zur Sputumprobe, das Zentrifugieren der Sputumprobe, bis sie in eine Überstand- und eine Pelletphase fraktioniert worden ist, und das Messen des Pellets.

12. Verfahren nach Anspruch 11, worin das Säugetierpatienten-Sputum von einem Patienten mit einer Erkrankung der Atmungsorgane, die mit infizierten Atemwegssekreten einhergeht, stammt.

13. Verfahren nach Anspruch 11, worin die Erkrankung der Atmungsorgane cystische Fibrose ist.

14. Verfahren nach Anspruch 11, worin die Erkrankung der Atmungsorgane Bronchitis ist.

15. Verfahren nach Anspruch 11, worin die Erkrankung der Atmungsorgane Lungenentzündung ist.

16. Verfahren nach Anspruch 11, worin das Therapeutikum rhDNase ist.

17. Verfahren nach Anspruch 11, worin das Therapeutikum ein Antibiotikum ist, das bei der Behandlung einer Erkrankung der Atmungsorgane nützlich ist.

18. Verfahren nach Anspruch 11, worin das Therapeutikum in einem Volumen, das 50% des Probengewichts des Sputums entspricht, zugegeben wird.

19. Verfahren zur Bestimmung der Kompaktion einer mit DNase behandelten Sputumprobe von einem Säugetierpatienten, der DNase-Therapie benötigt, umfassend das Abnehmen der Sputumprobe vom Säugetierpatienten, das Zugeben von DNase zur Sputumprobe, das Zentrifugieren des mit DNase behandelten Sputum, bis es in eine Überstand- und eine Pelletphase fraktioniert ist, und das Messen des Pellets.

20. Verfahren nach Anspruch 19, worin der Säugetierpatient ein Mensch ist und die DNase rekombinante menschliche DNase I (rhDNase I) ist.

21. Verfahren nach Anspruch 19, worin die rekombinante menschliche DNase I in einer Konzentration von zumindest 1 µg rhDNase I/ml Sputum vorliegt.

22. Verfahren nach Anspruch 19, worin die DNase in einem Volumen, das 50% des Gewichts des Sputums entspricht, zugegeben wird.

## Revendications

1. Procédé de mesure du compactage d'un échantillon de crachat prélevé sur un patient mammifère atteint d'une maladie respiratoire associée à des sécrétions de voies aériennes infectées, comprenant l'obtention d'un échantillon de crachat à partir d'un patient mammifère, la centrifugation dudit échantillon de crachat jusqu'à son fractionnement en une phase surnageante et une phase sous forme de culot, et la mesure du culot.

2. Procédé selon la revendication 1, dans lequel un diluant est ajouté à l'échantillon de crachat avant centrifugation.

3. Procédé selon la revendication 2, dans lequel le diluant est de l'eau ajoutée en volume égal à 50% du poids du crachat.

4. Procédé selon la revendication 1, dans lequel ledit crachat de patient mammifère provient d'un patient atteint de mucoviscidose.

5. Procédé selon la revendication 1, dans lequel ledit crachat de patient mammifère provient d'un patient atteint de pneumonie.

6. Procédé selon la revendication 1, dans lequel ledit crachat de patient mammifère provient d'un patient atteint de bronchite.

7. Procédé selon la revendication 1, dans lequel ledit crachat de patient mammifère provient d'un patient atteint d'infections du sinus.

8. Procédé selon la revendication 1, dans lequel ledit patient mammifère a reçu un traitement par un agent thérapeutique.

9. Procédé selon la revendication 1, dans lequel ledit patient mammifère a reçu un traitement par la DN-ase.

10. Procédé selon la revendication 1, dans lequel ledit patient mammifère a fait l'objet d'une physiothérapie thoracique.

11. Procédé de mesure du compactage d'un échantillon de crachat mammifère traité *in vitro* par un agent thérapeutique, comprenant l'obtention d'un échantillon de crachat à partir du patient mammifère, l'adjonction d'un agent thérapeutique à l'échantillon de crachat, la centrifugation dudit échantillon de crachat jusqu'à son fractionnement en une phase surnageante et une phase sous forme de culot, et la mesure du culot.

12. Procédé selon la revendication 11, dans lequel ledit crachat de patient mammifère provient d'un patient atteint d'une maladie respiratoire associée à des sécrétions de voies aériennes infectées.

13. Procédé selon la revendication 11, dans lequel ladite maladie respiratoire est la mucoviscidose.

14. Procédé selon la revendication 11, dans lequel ladite maladie respiratoire est une bronchite.

15. Procédé selon la revendications 11, dans lequel ladite maladie respiratoire est une pneumonie.

16. Procédé selon la revendication 11, dans lequel ledit agent thérapeutique est la rhDN-ase.

17. Procédé selon la revendication 11, dans lequel ledit agent thérapeutique est un antibiotique utile dans le traitement des maladies respiratoires.

18. Procédé selon la revendication 11, dans lequel ledit agent thérapeutique est ajouté à un volume égal à 50% du poids de l'échantillon de crachat.

19. Procédé de mesure du compactage d'un échantillon de crachat traité par la DN-ase prélevé sur un patient mammifère nécessitant une thérapie par la DN-ase comprenant, l'obtention d'un échantillon de crachat à partir du patient mammifère, l'addition de DN-ase à l'échantillon de crachat, la centrifugation du crachat traité par la DN-ase jusqu'à son fractionnement en une phase surnageante et une phase sous forme de culot, et la mesure du culot.

20. Procédé selon la revendication 19, dans lequel ledit patient mammifère est être humain et ladite DN-ase est une DN-ase I recombinante humaine (rhDN-ase I).

21. Procédé selon la revendication 19, dans lequel ladite DN-ase I humaine recombinante est en concentration d'au moins 1 µg de rhDN-ase I/ml de crachat.

22. Procédé selon la revendication 19, dans lequel ladite DN-ase est ajoutée à un volume égal à 50% en poids du crachat.


### SEQUENCE LISTING

(1) GENERAL INFORMATION:
   (i) APPLICANT: Genentech. Inc.
   (ii) TITLE OF INVENTION: Compaction Assay for Assessment of Respiratory Disease
      Therapy
   (iii) NUMBER OF SEQUENCES: 1
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 5.25 inch, 360 Kb floppy disk
      (B) COMPUTER: IBM PC compatible
      (C) OPERATING SYSTEM: PC-DOS/MS-DOS
      (D) SOFTWARE: patin (Genentech)
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME: Johnston, Sean A.
      (B) REGISTRATION NUMBER: 35,910
      (C) REFERENCE/DOCKET NUMBER: 792C1
   (ix) TELECOMMUNICATION INFORMATION:
      (A) TELEPHONE: 415/225-3562
      (B) TELEFAX: 415/952-9881
      (C) TELEX: 910/371-7168

   (2) INFORMATION FOR SEQ ID NO: 1:
   (i) SEQUENCE CHARACTERISTICS:
      (A) LENGTH: 1039 bases
      (B) TYPE: nucleic acid
      (C) STRANDEDNESS: single
      (D) TOPOLOGY: linear
   (xi) SEQUENCE DESCRIPTION: SEQ ID NO: 1:
   TCCTGCACAG GCAGTGCCTT GAAGTGCTTC TTCAGAGACC TTTCTTCATA 50
   GACTACTTTT TTTTCTTTAA GCAGCAAAAG GAGAAAATTG TCATCAAAGG 100
   ATATTCCAGA TTCTTGACAG CATTCTCGTC ATCTCTGAGG ACATCACCAT 150
   CATCTCAGG   ATG AGG GGC ATG AAG CTG CTG GGG GCG CTG 189
   CTG GCA CTG GCG GCC CTA CTG CAG GGG GCC GTG TCC CTG 228
   AAG ATC GCA GCC TTC AAC ATC CAG ACA TTT GGG GAG ACC 267
   AAG ATG TCC AAT GCC ACC CTC GTC AGC TAC ATT GTG CAG 306
   ATC CTG AGC CGC TAT GAC ATC GCC CTG GTC CAG GAG GTC 345
   AGA GAC AGC CAC CTG ACT GCC GTG GGG AAG CTG CTG GAC 384
   AAC CTC AAT CAG GAT GCA CCA GAC ACC TAT CAC TAC GTG 423
   GTC AGT GAG CCA CTG GGA CGG AAC AGC TAT AAG GAG CGC 462
   TAC CTG TTC GTG TAC AGG CCT GAC CAG GTG TCT GCG GTG 501
   GAC AGC TAC TAC TAC GAT GAT GGC TGC GAG CCC TGC GGG 540
   AAC GAC ACC TTC AAC CGA GAG CCA GCC ATT GTC AGG TTC 579
   TTC TCC CGG TTC ACA GAG GTC AGG GAG TTT GCC ATT GTT 618
   CCC CTG CAT GCG GCC CCG GGG GAC GCA GTA GCC GAG ATC 657
   GAC GCT CTC TAT GAC GTC TAC CTG GAT GTC CAA GAG AAA 696
   TGG GGC TTG GAG GAC GTC ATG TTG ATG GGC GAC TTC AAT 735
   GCG GGC TGC AGC TAT GTG AGA CCC TCC CAG TGG TCA TCC 774
   ATC CGC CTG TGG ACA AGC CCC ACC TTC CAG TGG CTG ATC 813
   CCC GAC AGC GCT GAC ACC ACA GCT ACA CCC ACG CAC TGT 852
   GCC TAT GAC AGG ATC GTG GTT GCA GGG ATG CTG CTC CGA 891
   GGC GCC GTT GTT CCC GAC TCG GCT CTT CCC TTT AAC TTC 930
   CAG GCT GCC TAT GGC CTG AGT GAC CAA CTG GCC CAA GCC 969
   ATC AGT GAC CAC TAT CCA GTG GAG GTG ATG CTG AAG TGAGC 1010
   AGCCCCTCCC CACACCAGTT GAACTGCAG 1039